(19) Europäisches Patentamt
European Patent Office
Office européen des brevets

(11) EP 1 032 559 B1

(12) **EUROPEAN PATENT SPECIFICATION**

(45) Date of publication and mention of the grant of the patent:
**29.11.2006 Bulletin 2006/48**

(21) Application number: **98945914.4**

(22) Date of filing: **04.09.1998**

(51) Int Cl.:
*C07D 207/06* (2006.01)

(86) International application number:
**PCT/US1998/018587**

(87) International publication number:
**WO 1999/016746 (08.04.1999 Gazette 1999/14)**

(54) **AMINOALKYLPHENOL DERIVATIVES FOR TREATING DEPRESSION AND MEMORY DYSFUNCTION**

AMINOALKYLPHENOL-DERIVATE ZUR BEHANDLUNG VON DEPRESSION UND GEDÄCHTNISFUNKTIONSSTÖRUNG

DERIVES D'AMINOALKYLPHENOLS POUR TRAITER LA DEPRESSION ET LE DYSFONCTIONNEMENT DE LA MEMOIRE

(84) Designated Contracting States:
**AT BE CH CY DE DK ES FI FR GB GR IE IT LI LU MC NL PT SE**

(30) Priority: **29.09.1997 US 939466**

(43) Date of publication of application:
**06.09.2000 Bulletin 2000/36**

(73) Proprietor: **Aventis Pharmaceuticals, Inc.**
**Bridgewater, New Jersey 08807 (US)**

(72) Inventors:
- **KOSLEY, Raymond, W., Jr.**
  **Bridgewater, NJ 08807 (US)**
- **PALERMO, Mark, G.**
  **Oro Valley, AZ 85737 (US)**
- **SHIMSHOCK, Stephen, J.**
  **Somerville, NJ 08876 (US)**
- **WOLF, Veronica**
  **Sandy, UT 84093 (US)**

(74) Representative: **Minoja, Fabrizio et al**
**Bianchetti Bracco Minoja S.r.l.**
**Via Plinio, 63**
**20129 Milano (IT)**

(56) References cited:

| | |
|---|---|
| **GB-A- 1 308 259** | **GB-A- 2 123 826** |
| **US-A- 2 927 924** | **US-A- 5 051 422** |
| **US-A- 5 143 910** | **US-A- 5 681 954** |

- **CASAGRANDE C ET AL: "SYNTHESIS AND CHEMICAL PROPERTIES OF IBOPAMINE AND OF RELATED ESTEROF N-SUBSTITUTED DOPAMINES - SYNTHESIS OF IBOPAMINE METABOLITES" ARZNEIMITTEL FORSCHUNG. DRUG RESEARCH, EDITIO CANTOR. AULENDORF, DE, vol. 36, no. 2A, 1986, pages 291-303, XP000926659 ISSN: 0004-4172**
- **CHEMICAL ABSTRACTS, Vol. 54, No. 6, 25 March 1960, (Columbus, Ohio, USA), page 5678, Abstract No. 5679b, YAMAZAKI T., "Synthesis in the Azabenzoquinolizine Group II", XP002916040; & YAKAGAKU ZASSHI, 79, (1959), 1003-8.**
- **CHEMICAL ABSTRACTS, Vol. 54, No. 14, 25 July 1960, (Columbus, Ohio, USA), page 14280, MILLS J., "Phenethyl Substituted Piperazines", XP002916041; & US,A,2 927 924 (08 March 1960).**
- **CHEMICAL ABSTRACTS, Vol. 59, No. 11, 25 November 1963, (Columbus, Ohio, USA), page 12804, BOISSIER J.R. et al., "Synthesis and Phamacological Study of New Piperazine Derivatives", XP002916042; & J. MED. CHEM., 6 (5), (1963), 541-4.**
- **CHEMICAL ABSTRACTS, Vol. 62, No. 5, 01 March 1965, (Columbus, Ohio, USA), page 5277, Abstract No. 5277g, RATOUIS R. et al., "Synthesis and Pharmacological Study of New Piperazine Derivatives Phenethylpiperazines", XP002916043; & J. MED. CHEM., 8(1), (1965), 104-7.**

EP 1 032 559 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- CHEMICAL ABSTRACTS, Vol. 62, No. 12, 07 June 1965, (Columbus, Ohio, USA), page 15243, Abstract No. 15243g, MULL R.P. et al., "N,N'-Disubstituted Compounds with Diverse Biological Activities", XP002916044 & J. MED. CHEM., 8(3), (1965), 332-8.
- CHEMICAL ABSTRACTS, Vol. 62, No. 13, 21 June 1965, (Columbus, Ohio, USA), page 16566, FULLER R., "Inhibition of Rat Liver Tryptophan Hydroxylase In Vitro", XP002916045; & LIFE SCI., 4(1), (1965), 1-6.
- CHEMICAL ABSTRACTS, Vol. 63, No. 11, 22 November 1965, (Columbus, Ohio, USA), page 14880, "Pyrimidine Derivatives. Science Union et Cie.-Societe Francaise de Recherche Medicale", XP002916046; & NL,A,64 13349 (20 May 1965).
- CHEMICAL ABSTRACTS, Vol. 70, No. 3, 20 January 1969, (Columbus, Ohio, USA), page 286, Abstract No. 11676w, PRASAD R.N. et al., "Potential Antihypertensive Agents II Unsymmetrically 1,4-Disubstituted Piperazines", XP002916047; & J. MED. CHEM., 1968, 11(6), 1144-50 (Eng).
- CHEMICAL ABSTRACTS, Vol. 97, No. 15, 11 October 1982, (Columbus, Ohio, USA), page 728, Abstract No. 127638x, BLUME E. et al., "1-(1,3-Dioxolan-2-Ylmethyl)Azoles, their Salts and their Use", XP002916048; & EP,A,50 298 (28 April 1982).
- CHEMICAL ABSTRACTS, Vol. 115, No. 17, 28 October 1991, (Columbus, Ohio, USA), page 921, Abstract No. 183239v, KATRITZKY A.R. et al., "Compounds with Potential Second Order Non-Linear Optical Activity", XP002916049; & HETEROCYCL. CHEM., 1991, 28(4), 1115-1119 (Eng).
- CHEMICAL ABSTRACTS, Vol. 53, No. 9, 10 May 1959, (Columbus, Ohio, USA), page 9254, THOMAE K. et al., "Tertiary Amines", XP002916050; & DE,A,963 424 (09 May 1957).
- CHEMICAL ABSTRACTS, Vol. 92, No. 19, 12 May 1980, (Columbus, Ohio, USA), page 584, Abstract No. 163771r, KLAUSE R. et al., "Neuropsychotropic Activity of Dopamine Analogous 4,7-Methano-1H-Isoindoles", XP002916051; & ARCH. PHERM., 1979, 312(12), 982-94 (Ger).
- CHEMICAL ABSTRACTS, Vol. 71, No. 7, 18 August 1969, (Columbus, Ohio, USA), page 258, Abstract No. 30163p, MNDZHOYAN A.L. et al., "Synthesis of P-Alkoxybenzoic Acid Derivatives XXV Heterocyclically Substitute Amides of 3,4-Dialkoxybenzoic Acids and the Corresponding Amines", XP002916052; & ARM. KHIM. ZH., 1968, 21(9), 783-6 (Russ).
- CHEMICAL ABSTRACTS, Vol. 61, No. 2, 20 July 1964, (Columbus, Ohio, USA), page 2331, SCHUELTZ E. et al., "The Relation Between Chemical Constitution and Pharmacologic Activity of 1-Phenyl-1-Amino Alkanes", XP002916053; & ARZNEIMITTEL-FORSCH., 14, (1964), 178-86.
- CHEMICAL ABSTRACTS, Vol. 106, No. 7, 16 February 1987, (Columbus, Ohio, USA), page 594, Abstract No. 49684x, CASAGRANDE C. et al., "Synthesis and Chemical Properties of Ibopamine and of Related Esters of N-Substituted Dopamines - Synthesis of Ibopamine Metabolites", XP002916054; & ARZNEIM.-FORSCH., 1986, 36(2A), 291-303 (Eng).
- CHEMICAL ABSTRACTS, Vol. 77, No. 9, 28 August 1972, (Columbus, Ohio, USA), page 468, Abstract No. 61607q, KAISER A. et al., "(3,4-Dihydroxyphenethyl)Amine Derivatives" XP002916055; & DE,A,2 153 801 (4 May 1972).
- CHEMICAL ABSTRACTS, Vol. 51, No. 22, 25 November 1957, (Columbus, Ohio, USA), page 18343, VOTAVA Z. et al., "Pharmacological Research on Synthetic Uterotonics II Substituted N-Benzylpiperidines and 3,4-Dimethoxybenzyl-amines", XP002916056; & PHYSIOL. BOHEMOSLOV., 3, (1954), 426-31.
- CHEMICAL ABSTRACTS, Vol. 56, No. 2, 22 January 1962, (Columbus, Ohio, USA), page 1324, KINDLER K. et al., "Mechanism of Chemical Reactions. XIX. Hydrogenation and Specific Hydrogenation by Bound Hydrogen", XP002916057; & ANN., 644, (1961),23-30.
- CHEMICAL ABSTRACTS, Vol. 56, No. 6, 19 March 1962, (Columbus, Ohio, USA), page 6012, BATTERSBY A.R. et al., "Synthetic Applications of 1,2-Dihydroisoquinolines", XP002916058; & TETRAHEDRON, 14, (1961), 46-53.
- CHEMICAL ABSTRACTS, Vol. 85, No. 19, 08 November 1976, (Columbus, Ohio, USA), page 503, Abstract No. 142879x, HEEP U. et al., "Insect-Repellant Carbamates", XP002916059; & DE,A,2 456 279 (12 August 1976).
- CHEMICAL ABSTRACTS, Vol. 51, No. 20, 25 October 1957, (Columbus, Ohio, USA), page 15699, AKIYA S. et al., "Synthetic Compounds Active Against Salmonella- Dysentery Group Bacili", XP002916060; & JAPAN J. EXPTL. MED., 26, (1956), 91-112.
- CHEMICAL ABSTRACTS, Vol. 64, No. 4, 14 February 1966, (Columbus, Ohio, USA), page 5197, YAMADA S. et al., "Solvent Effects on the Optical Rotatory Dispersion of N-Thiacyl Derivatives of AlphaAmino Acids", XP002916061; & CHEM. PHARM. BULL., 13(10), (1965), 1266-1269 (Eng).




(Cont. next page)

- **CHEMICAL ABSTRACTS, Vol. 123, No. 21, 20 November 1995, (Columbus, Ohio, USA), page 1174, Abstract No. 285700w, STEEN E. et al., "Synthesis of 3-Acyl- and 3-Carbamoyl Flavones", XP002916062; & ACTA. CHEM. SCAND., 1995, 49(7), 524-9 (Eng).**
- **CHEMICAL ABSTRACTS, Vol. 123, No. 3, 17 July 1995, (Columbus, Ohio, USA), page 830, Abstract No. 32860e, EDGAR V. et al., "New Bis(Hispidine) Derivatives", XP002916063; & DE,A,4 332 203 (23 March 1995).**

## Description

The present invention relates to aminoalkylphenols. More particularly, the present invention relates to aminoalkylphenol derivatives of formula 1

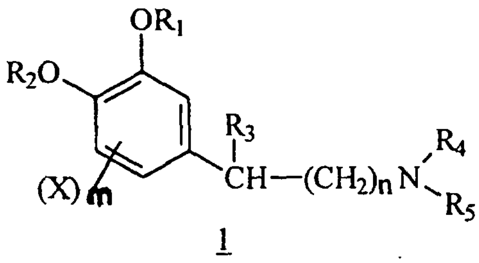

1

wherein:

$R_1$ is a group of the formula $CH_2C{\equiv}C\text{-}R_9$;

$R_2$ is hydrogen, loweralkyl, a group of the formula $CONP_6R_7$, a group of the formula $SO_2R_{10}$, a group of the formula

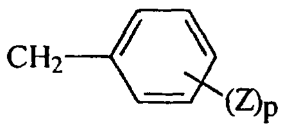

or a group of the formula $Si(R_{11})_3$;

$R_3$ is hydrogen or loweralkyl;

$R_4$ and $R_5$ taken together with the nitrogen atom to which they are attached form a group of the formula

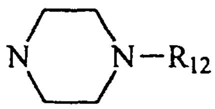 ;

$R_6$ is hydrogen or loweralkyl;

$R_7$ is alkyl of 1 to 10 carbon atoms, a group of the formula

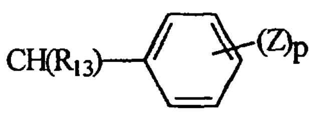 ,

a group of the formula

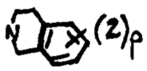 ,

or a group of the formula

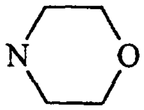 ;

$R_6$ and $R_7$ taken together with the nitrogen atom to which they are attached form a group of the formula

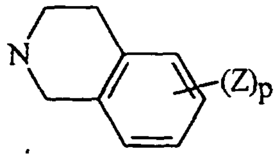 ;

$R_9$ is hydrogen, a group of the formula $C(R_{14}R_{14'})OH$, a group of the formula

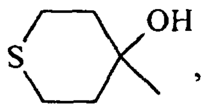 ,

or a group of the formula

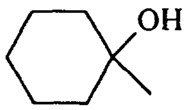 ;

$R_{10}$ is loweralkyl;

$R_{11}$ is loweralkyl;

$R_{12}$ is loweralkyl, hydroxyloweralkyl, a group of the formula $COR_{15}$, a group of the formula

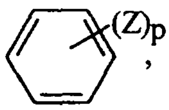 ,

a group of the formula

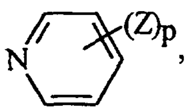 ,

a group of the formula

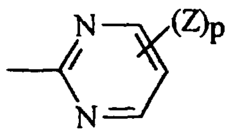 ,

a group of the formula

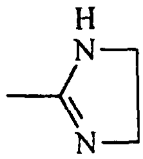

,

or a group of the formula

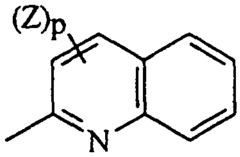

;

$R_{13}$ is hydrogen or loweralkyl;

$R_{14}$ is hydrogen or loweralkyl;

$R_{14'}$ is hydrogen or loweralkyl;

$R_{15}$ is a group of the formula

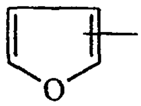

or loweralkyl;

$R_{20}$ is loweralkyl;

X is hydrogen or halogen;

Z is hydrogen, halogen, loweralkyl, hydroxyl, loweralkoxy, trifluoromethyl, nitro or cyano, $R_{20}CO$, or a group of the formula $OCONR_6R_7$;

m is 1 or 2;

n is 0 or 1;

p is 1 or 2;

the optical isomers thereof; or the pharmaceutically acceptable salts thereof, which are useful for relieving memory dysfunction and thus indicated in the treatment of Alzheimer's disease, alone, or in combination with adjuvants.

Subgeneric thereto are compounds wherein:

(a) $R_2$ is loweralkyl;
(b) $R_2$ is a group of the formula $CONR_6R_7$;
(c) $R_2$ is a group of the formula $CONR_6R_7$ and $R_4$ and $R_5$ taken together with the nitrogen to which they are attached form a group of the formula

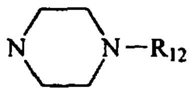 ;

and n is 0.

As used throughout the specification and appended claims, the term "alkyl" refers to a straight or branched chain hydrocarbon radical containing no unsaturation and having 1 to 12 carbon atoms. Examples of alkyl groups are methyl, ethyl, 1-propyl, 2-propyl, 1-butyl, 1-pentyl, 3-hexyl, 4-heptyl, 2-octyl, 3-nonyl, 4-decyl, undecyl, dodecyl, and the like. The term "alkanol" refers to a compound formed by a combination of an alkyl group and hydroxy radical. Examples of alkanols are methanol, ethanol, 1- and 2-propanol, 2,2-dimethylethanol; hexanol, octanol, decanol and the like. The term "alkanoic acid" refers to a compound formed by combination of a carboxyl group with a hydrogen atom or alkyl group. Examples of alkanoic acids are formic acid, acetic acid, propanoic acid, 2,2-dimethylacetic acid, hexanoic acid, octanoic acid, decanoic acid and the like. The term "halogen" refers to a member of the family fluorine, chlorine, bromine, or iodine. The term "alkanoyl" refers to the radical formed by removal of the hydroxyl function from an alkanoic acid. Examples of alkanoyl groups are formyl, acetyl, propionyl, 2,2-dimethylacetyl, hexanoyl, octanoyl, decanoyl and the like. The term "alkoxy" refers to a monovalent substituent which consists of an alkyl group linked through an ether oxygen and having its free valence from the ether oxygen. Examples of alkoxy groups are methoxy, ethoxy, proproxy, 2,2-dimethylethoxy, hexoxy, octoxy, decoxy and the like. The term "lower" as applied to any of the aforementioned groups refers to a group having a carbon skeleton containing up to and including 10 carbon atoms.

The compounds of the present invention which lack an element of symmetry exist as optical antipodes and as the racemic forms thereof. The optical antipodes may be prepared from the corresponding racemic forms by standard optical resolution techniques, involving, for example, the separation of diastereomeric salts of those instant compounds characterized by the presence of a basic amino group and an optically active acid, those instant compounds characterized by the presence of a carboxylic acid group and an optically active base, or by synthesis from optically active precursors.

The present invention comprehends all optical isomers and racemic forms thereof of the compounds disclosed and claimed herein. The formulas of the compounds shown herein are intended to encompass all possible optical isomers of the compounds so depicted.

The novel aminoalkylphenols of the present invention are prepared by the processes illustrated in the Reaction Schemes. To prepare an aminoalkylphenol 1 wherein $R_3$ is hydrogen or loweralkyl and n is 0, a benzaldehyde 6, or a phenylalkylketone 10, is condensed with an amine 7 in the presence of a reducing agent to provide 8 or 11, respectively. The reductive amination is generally performed in the presence of a mild selective reducing agent such as an alkali metal trialkanoyloxyborohydride or an alkali metal cyanoborohydride in a suitable solvent. Among alkali metal trialkanoyloxy- borohydrides, there may be mentioned lithium-, sodium- and potassium triacetoxyborohydride. Among alkali metal cyanoborohydrides, there may be mentioned lithium-, sodium- and potassium cyanoborohydride. Among suitable solvents, there may be mentioned halocarbons such as dichloromethane, trichloromethane, 1,2-dichloroethane and 1,1-dichloroethane, or ethereal solvents such as tetrahydrofuran, dioxane and 2-methoxyethyl ether, optionally containing an alkanoic acid such as acetic acid when a trialkanoyloxyborohydride is employed, and an alkanol such as methanol or ethanol in the presence of a mineral acid such as hydrochloride acid or an alkanoic acid such as acetic acid when a cyanoborohydride is employed. Sodium triacetoxyborohydride is the preferred reducing agent; 1,2-dichloroethane is the preferred halocarbon. The reaction of a benzaldehyde 6 and a secondary amine 7 is preferably carried out in the absence of an alkanoic acid such as acetic acid. While the temperature at which the reaction is conducted is not narrowly critical, the reaction is conveniently carried out at ambient temperature.

Alternatively, an aminoalkylphenol 1 wherein $R_3$ is hydrogen and n is 0, is prepared by condensing a benzyl halide 9 with an amine 7 to provide 13. The condensation is accomplished by means of an alkali metal hydride such as sodium hydride in a halocarbon such as chloromethane, dichloromethane, or 1,1- or 1,2-dichloroethane or dimethylformamide, at about ambient temperature, although reduced or elevated temperatures may be employed. The preferred condensation medium is sodium hydride as a dispersion in oil in dichloromethane.

To fabricate an aminoalkylphenol derivative 1 wherein $R_3$ is hydrogen or loweralkyl and n is 1, a haloethylphenol 12 is condensed with an amine 7 to provide 13 by the processes herein described.

To gain access to an aminoalkylphenol derivative 1 wherein $R_3$ is loweralkyl and n is 0, a phenylalkylketone 10 is condensed with an amine 7 to provide 11. The reaction is carried out in the presence of a reducing agent such as titanium (IV) alkoxide in a suitable solvent such as acetonitrile or dichloromethane at about ambient temperature followed by an alkali metal cyanoborohydride in an alkanol, or an alkanoic acid such as acetic acid or a mineral acid such as hydrochloric acid, optimally an alkanoic acid also at ambient temperature. Among titanium (IV) alkoxides there may be mentioned titanium (IV) methoxide, titanium (IV) ethoxide, titanium (IV) 2-propoxide, and titanium (IV) 1-propoxide.

Among alkali metal cyanoborohydrides there may be mentioned lithium cyanoborohydride, sodium cyanoborohydride and potassium cyanoborohydride. Among alkanols, there may be mentioned methanol, ethanol, 1-propanol and 2-propanol. The preferred reagents for effecting the reductive condensation are titanium (IV) 2-propoxide, sodium cyanoborohydride, ethanol and dichloromethane.

The aminoalkylphenol derivatives and related compounds thereof of the present invention of formula 1 having the described functionality on the benzene ring and in the side-chain, i.e., compounds of formula 1 wherein $R_1$,$R_2$, $R_3$, $R_4$, $R_5$, X, m and n are as hereinbefore disclosed may be prepared starting from benzaldehydes 6, phenylalkyl ketones 10, benzylhalides 9, or phenylethylhalides 12 having the described functionality intact or from the aminoalkylphenol derivatives 8, 11, or 13 by manipulation of the functionality thereof.

To convert an aromatic hydroxyl group (-OH) to a carbamoyloxy moiety

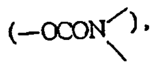

an aminoalkylphenol 1 wherein $R_2$ is hydrogen is treated with, for example, a carbamoyl halide 14 of the formula

$R_6R_7NCOHal$ 14

wherein $R_6$ and $R_7$ are as herein defined and Hal is bromo or chloro in the presence of an acid acceptor such as an alkali metal carbonate, e.g., lithium-, sodium-, potassium- or cesium carbonate in a suitable solvent such as acetonitrile or dichloromethane, or combinations thereof, at about ambient temperature. Cesium carbonate is the preferred acid acceptor.

Alternatively, a tertiary amine such as 1,8-diazabicyclo[5.4.0]undec-7-ene may be used as the acid acceptor and acetonitrile as the solvent in the reaction of a phenol 1 with a carbamoyl halide 14.

The conversion of a hydroxyl group (-OH) to a carbamoyloxy moiety

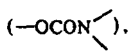

may also be effected by treating an aminoalkylphenol 1 wherein $R_2$ is hydrogen with an isocyanate 15

$R_6$ or $R_7$-N=C=O 15

wherein $R_6$ or $R_7$ is as herein defined in the presence of a copper (I) halide, wherein the halide is chloro or bromo, in ethyl acetate, acetonitrile or dichloromethane, or combinations thereof. The introduction of a carbamoyl group

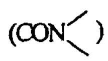

by means of an isocyanate 15 may also be accomplished in an ethereal solvent such as tetrahydrofuran in the presence of an alkali metal carbonate such as potassium carbonate. In addition, the modification of a hydroxyl group (-OH), i.e., the conversion to a carbamoyloxy function

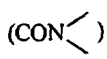

may be effected using an amine 16

$HNR_6R_7$ 16

wherein $R_6$ and $R_7$ are as defined herein in the presence of 1,1∂-carbonyldiimidazole in tetrahydrofuran.

To prepare aminoalkylphenols of formula 1 wherein $R_1$ is a group of the formula $CH_2C{\equiv}CR_9$ wherein $R_9$ is as described herein, i.e., to introduce the ethynylalkyl moiety ($CH_2C{\equiv}C$-) into the phenoxy system, an ethynylmethoxybenzaldehyde 20, which is fabricated from a benzaldehyde 17 wherein $R_1$ is loweralkyl and X and m are as herein described by sulfonylation of 17 to an alkylsulfonylbenzaldehyde 18 wherein $R_1$ and $R_{15}$ are loweralkyl, dealkylation of 18 to a hydroxybenzaldehyde 19 followed by ethynylalkylation of 19 to ethynylalkoxybenzaldehyde 20, is converted to an aminoalkylbenzene 21 wherein $R_3$, $R_4$, $R_5$ and n are as described herein, which is in turn hydrolyzed to an aminoalkylphenol 22 and carbamoylated to carbamate 23 wherein $R_6$ and $R_7$ are as described herein. The conversion of an ethynylalkoxybenzaldehyde 20 to an aminoalkylbenzene 21 is accomplished by the methods disclosed herein. The hydrolysis of a sulfonyloxybenzene 21 to a phenol 22 is performed in an aqueous alkanol, such as aqueous methanol, containing an alkali metal hydroxide such as sodium hydroxide within a temperature range of about 25° to 75° C, a hydrolysis temperature of about 50° C being preferred. The carbamoylation of 22 to 23 is effected as herein described. Alternatively, a phenol 22 is converted to carbamate 23 by treatment with an alkali metal carbonate such as potassium carbonate in an ethereal solvent such as tetrahydrofuran followed by an isocyanate of formula 15 at ambient temperature as herein described.

Substituted ethynylmethoxybenzenecarbamates 26 wherein $R_9$ is a group as described herein other than hydrogen are prepared from ethynylmethoxyphenols 22 by protecting the phenolic group thereof, introducing the $R_9$ moiety to yield substituted ethynylmethoxybenzenes 25, removing the protecting group of 25 and elaborating the carbamoyloxybenzene 26 wherein $R_6$ and $R_7$ are as herein described.

The protection of the phenolic group of ethynylmethoxyhydroxybenzene 22 is accomplished by treating the phenol 22 with tri-(2-propyl)silyl chloride of formula 27a

$((CH_3)_2CH)_3SiCl$ 27a

in a dipolar aprotic solvent. e.g., dimethylacetamide, dimethylformamide, hexamethylphosphoramide or dimethylsulfoxide, dimethylformamide being preferred, in the presence of an acid acceptor such as a tertiary amine, e.g., di-(2-propyl) ethylamine at ambient temperature.

The substituent ($R_9$), i.e., a group of the formula ($R_{14}R_{14'}$) CHOH wherein $R_{14}$ is as described herein, or a group of the formula

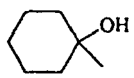

,

or a group of the formula

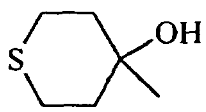

,

is introduced by treating the ethynylmethoxysilyloxybenzene 24 with an alkyllithium such as n-butyllithium in an ethereal solvent such as tetrahydrofuran at a reduced temperature within the range of about -30° to about -50°C followed by a carbonyl compound of formula 28a

$(R_{14}R_{14'})C{=}O$ 28a

wherein $R_{14}$ and $R_{14}$, are hydrogen or loweralkyl, or a compound of the formula

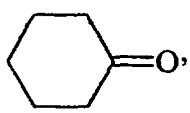

or a compound of the formula

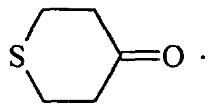

A carbamoyloxy derivative 26 of a substituted ethynylmethoxybenzene 25 is prepared in situ by treatment of 25 with tetra-n-butylammonium fluoride in an ethereal solvent such as tetrahydrofuran at ambient temperature to remove the protecting group followed by an isocyanate of formula 15 in the presence of a lithium halide, preferably lithium chloride.

Similarly, to prepare aminoalkylphenols of formula 1, i.e., compounds of formula 30 wherein $R_9$ is as herein described, a hydroxybenzaldehyde 27 is ethynylalkylated to afford ethynylalkoxyaldehyde 28 which is reductively aminated to an aminoalkylethynylalkoxybenzene 30 and then alkylated to an ethynylalkoxy carbinol 30. The sequence of reactions is performed by processes herein described.

Removal of functional groups bound to the potential phenolic oxygen atoms of the aminoalkylbenzenes of the present invention, i.e., the formation of aminoalkylphenols of formulas 36 and 37 is effected by hydrolysis, debenzylation and/or demethylation processes. For example, to remove an aminocarbonyl group of a compound of formula 35 wherein $R_1$ is a group of the formula $CONR_6R_7$ wherein $R_6$ and $R_7$ are as hereinbefore described and $R_2$ is hydrogen, loweralkyl or benzyl, a carbamate 35 is treated with an alkali metal hydroxide in an alkanol at ambient temperature. Among alkali metal hydroxides there may be mentioned lithium, sodium or potassium hydroxide. Among alkanols there maybe mentioned methanol, ethanol or 1-propanol. Sodium hydroxide in methanol is the preferred alkali metal hydroxide; methanol is the preferred alkanol.

Similarly, removal of an alkanoyl group from an aminoalkylbenzene 35 wherein $R_2$ is a group of the formula RCO wherein R is loweralkyl and $R_1$ is hydrogen, loweralkyl or benzyl, is also effected by hydrolysis. In this case, the hydrolysis is achieved by treating the ester 35 with alkali metal hydroxide such as sodium hydroxide and an alkanol such as aqueous ethanol at a slightly elevated temperature of about 50° C, although a hydrolysis temperature within the range of about ambient temperature to about the reflux temperature of the reaction medium is suitable.

To remove a benzyl group from a compound of formula 35, i.e., to debenzylate an aminoalkylbenzene 35 wherein $R_2$ is benzyl and $R_1$ is loweralkyl, an aminoalkylbenzene 35 is treated with ferric chloride in a halocarbon such as dichloromethane at the reflux temperature of the reaction mediums or with hydrogen in the presence of a metal catalyst such as palladium in a solvent such as acetic acid or methanol.

To remove an alkoxy group from a compound of formula 35, i.e., to dealkylate an aminoalkylbenzene 35 wherein $R_2$ is loweralkyl, an aminoalkylbenzene 35 is treated with a hydrohalic acid such as hydrobromic acid at an elevated temperature of about 100°.

To prepare aminoalkylcarbamates 48, an aminoalkylphenol 47 is converted to carbamate 48 by methods hereindescribed.

REACTION SCHEME A

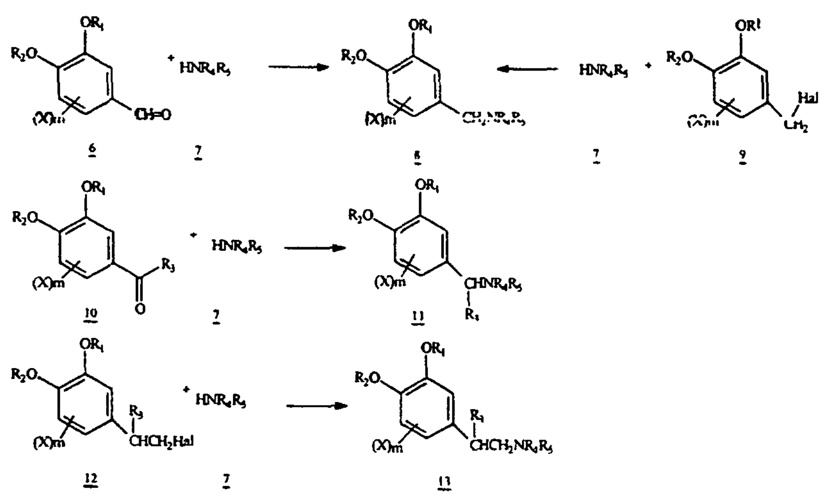

wherein $R_1$, $R_2$, $R_3$, $R_4$, $R_5$, X, and m are as hereinbefore described and Hal is bromo or chloro.

REACTION SCHEME B

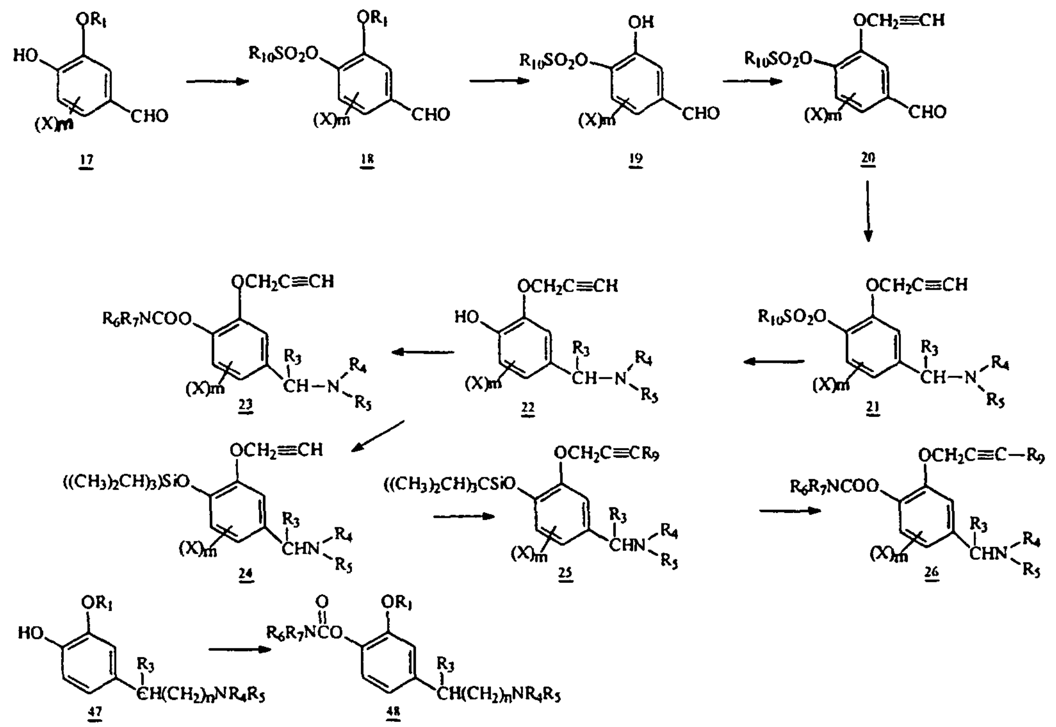

17 18 19 20 21 22 23 24 25 26 47 48

wherein $R_1$, $R_2$, $R_3$, $R_4$, $R_5$, $R_6$, $R_7$, $R_{10}$ and n are as hereinbefore described

REACTION SCHEME C

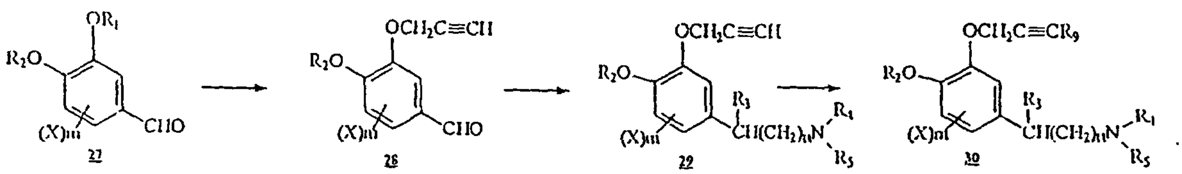

27 28 29 30

wherein $R_2$ is hydrogen, loweralkyl or benzyl, and $R_3$, $R_4$, $R_5$, X, m and n are as hereinbefore described.

REACTIONS CHEMED

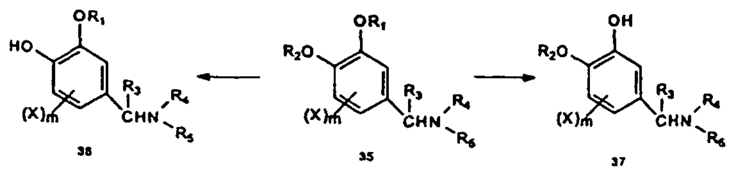

38 35 37

wherein $R_1$, $R_2$, $R_3$, $R_4$, $R_5$, X, m and n are as hereindescribed

The aminoalkylphenol derivatives of the present invention are useful as agents for the relief of memory dysfunction, particularly dysfunctions associated with decreased cholinergic activity such as those found in Alzheimer's disease. Relief of memory dysfunction activity is demonstrated in the in vitro inhibition of acetylcholinesterase assay,

an assay for the determination of the ability of a drug to inhibit the inactivation of acetylcholine, a neurotransmitter implicated in the etiology of memory dysfunction and Alzheimer's dementia. In this assay, a modification of a test described G.L. Ellman, et al., Biochemical Pharmacology, 7, 88 (1961), the following reagents are prepared and employed:

1. 0.05M Phosphate Buffer (pH 7.2)

A solution of monobasic sodium phosphate monohydrate (6.85 g) in distilled water (100 ml) is added to a solution of dibasic sodium phosphate heptahydrate (13.4 g) and distilled water (100 ml) until a pH of 7.2 was attained. The solution was diluted 1 to 10 with distilled water.

2. Substrate in Buffer

The 0.05M Phosphate Buffer (pH 7.2) was added to acetylthiocholine (198 mg) to a total volume of 100 ml, i.e., a quantity sufficient (gs) to 100 ml.

3. 5,5-Dithiobisnitrobenzoic acid in Buffer

The 0.05M Phosphate Buffer (pH 7.2) was added to 5.5-dithiobisnitrobenzoic acid to a total volume of 100 ml, i.e., a quantity sufficient (gs) to 100 ml.

4. Stock Solution of Drug

A 2 millimolar stock solution of the test drug is prepared in a quantity sufficient of either acetic acid or dimethyl sulfoxide to volume with 5,5-dithiobisnitrobenzoic acid in Buffer. Stock Solution of Drug is serially diluted (1:10) so that the final cuvette concentration is $10^{-4}$ molar.

Male Wistar rats are decapitated, brains rapidly removed, corpora striata dissected free, weighted and homogenized in 19 volumes (approximately 7 mg protein/ml) of 0.05M Phosphate Buffer (pH 7.2) using a Potter-Elvejhem homogenizer. A 25 µl aliquot of this suspension is added to 1 ml of the vehicle or various concentrations of the test drug and preincubated for 10 minutes at 37°C. Enzyme activity is measured with a Beckman DU-50 spectrophotometer with the following software and instrument settings:

1. Kinetics Soft-Pac™ Module #598273;
2. Program #6 Kindata;
3. Source - Vis;
4. Wavelength - 412 nm;
5. Sipper - none;
6. Cuvettes - 2 ml cuvettes using auto 6-sampler;
7. Blank - 1 for each substrate concentration;
8. Interval time - 15 seconds (15 or 30 seconds for kinetics);
9. Total time - 5 minutes (5 to 10 minutes for kinetics);
10. Plot - yes;
11. Span - autoscale;
12. Slope - increasing;
13. Results - yes (gives slope); and
14. Factor - 1.

Reagents are added to the blank and sample cuvettes as follows:

| | | |
|---|---|---|
| 1. | Blank: | 0.8 ml 5.5-Dithiobisnitrobenzoic Acid<br>0.8 ml Substrate in Buffer |
| 2. | Control: | 0.8 ml 5.5-Dithiobisnitrobenzoic Acid/Enzyme<br>0.8 ml Substrate in Buffer |
| 3. | Drug: | 0.8 ml 5.5-Dithiobisnitrobenzoic Acid/Drug/ Enzyme<br>0.8 ml Substrate in Buffer |

Blank values are determined for each run to control for non-enzymatic hydrolysis of substrate and these values

are automatically subtracted by the Kindata program available on kinetics soft-pac module. This program also calculates the rate of absorbance change for each cuvette.

For $IC_{50}$ Determinations

Substrate concentration is 10 millimolar diluted 1:2 in assay yielding final concentration of 5 millimolar. 5,5-dithiobisnitrobenzoic acid concentration is 0.5 millimolar yielding 0.25 millimolar final concentration.

$$\% \text{ Inhibition} = \frac{\text{Slope Control} - \text{Slope drug}}{\text{Slope Control}} \times 100$$

$IC_{50}$ values are calculated from log-probit analysis

TABLE I

| Compound | Inhibition of Acetylcholinesterase Activity $IC_{50}$(μM) |
|---|---|
| 4-(methylaminocarbonyloxy)-3-(propargyloxy) pyrrolidinomethylbenzene (reference) | 0.0036 |
| 4-methoxy-3-(propargyloxy)-1-[[4-(pyridin-2-yl) piperazin-1-yl] methyl]benzene | 26.9 |
| tacrine (reference) | 0.31 |

Relief of memory dysfunction is achieved when the present alkylaminophenol derivatives are administered to a subject requiring such treatment as an effective oral, parenteral or intravenous dose of from 0.10 to 50 mg/kg of body weight per day. A particularly effective amount is about 10 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.

The alkylaminophenol derivatives of the present invention are also useful as agents for treating depression. Depression treatment is demonstrated in the in vitro clonidine binding: $\alpha_2$-receptor assay, an assay for the determination of the ability of a drug to bind the clonidine: $\alpha_2$-receptor, performed by a modification of assays described by D. C. U'Prichard, et al., Molecular Pharmacology, 16, 47 (1979) and D. C. U'Prichard, et al., Molecular Pharm acology, 13, 454 (1976).

The following reagents are prepared:

1. Tris buffer, pH 7.7

   a. 57.2 g Tris hydrochloride 16.2 g Tris Base - q.s. to 1 liter (0.5 M Tris buffer, pH 7.7)
   b. Make a 1:10 dilution in distilled $H_2O$ (0.05 M Tris buffer, pH 7.7)

2. Tris buffer containing physiological ions
a. Stock Buffer

| | |
|---|---|
| Sodium chloride | 7.014 g |
| Potassium chloride | 0.372 g |
| Calcium chloride | 0.222 g - q.s. to 100 ml in 0.5 Tris buffer |
| Magnesium chloride | 0.204 g |

b. Dilute 1:10 in distilled $H_2O$. This yields 0.05 M Tris hydrochloride pH 7.7; containing sodium chloride (120 mM), potassium chloride (5 mM), calcium chloride (2 mM) and magnesium chloride (1 mM).

3. [4-$^3$H]-Clonidine Hydrochloride (20-30 Ci/mmol) is obtained from New England Nuclear. For $IC_{50}$ determinations: $^3$H-Clonidine is made up to a concentration of 120 nM and 50 μl added to each tube (yields a final concentration of 3 nM in the 2 ml volume assay).
4. Clonidine hydrochloride is obtained from Boehringer Ingelheim. A stock solution of 0.1 mM clonidine is made up to determine nonspecific binding. This yields a final concentration of 1 μM in the assay (20 μl to 2 ml).
5. Test compounds. For most assays, a 1 mM stock solution is made up in a suitable solvent and serially diluted, such that the final concentration in the assay ranges from $10^{-5}$ to $10^{-8}$M. Seven concentrations are used for each assay and higher or lower concentrations may be used, depending on the potency of the drug.

B. Tissue Preparation

Male Wistar rats are sacrificed by decapitation and the cortica tissue rapidly dissected. The tissue is homogenized in 50 volumes of 0.05 M Tris buffer, pH 7.7 (buffer 1b) with the Brinkman Polytron, then centrifuged at 40,000 g for 15 minutes. The supernatant is discarded and the pellet rehomogenized in the original volume of 0.05 M Tris buffer, pH 7.7 and recentrifuged as before. The supernate is discarded and the final pellet rehomogenized in 50 volumes of Buffer 2b. This tissue suspension is then stored on ice. The final tissue concentration is 10 mg/ml. Specific binding is 1% of the total added ligand and 80% of total bound ligand.

C. Assay

100 μl 0.5 M Tris-physiological salts, pH 7.7 (buffer 2a)

| | |
|---|---|
| 830 μl | Water |
| 20 μl | Vehicle (for total binding) or 0.1 mM clonidine (for nonspecific binding) or appropriate drug concentration |
| 50 μl | $^3$H-clonidine stock |
| 1000 μl | Tissue suspension |

Tissue homogenates are incubated for 20 minutes at 25° C with 3 nM $^3$H-clonidine and varying drug concentrations, then immediately filtered under reduced pressure on Whatman GF/B filters. The filters are washed with three five ml volumes of ice-cold 0.05 M Tris buffer, pH 7.7, then transferred to scintillation vials. Ten ml of liquescent counting solution is added to each sample which is then counted by liquid scintillation spectroscopy. Specific clonidine is defined as the difference between total bound and that performed using log-probit analysis. The percent inhibition at each drug concentration is the mean of triplicate determinations.

TABLE II

| Compound | Inhibition of clonidine binding activity $IC_{50}$ (μM) |
|---|---|
| 4-methoxy-3-(propargyloxy)-1-[[4-(pyridin-2-yl)piperazin-1-yl] methyl]benzene | 0.0346 |
| amitriptyline (reference) | 0.039 |

Depression treatment is achieved when the present aminoalkylphenol derivatives are administered to a subject requiring such treatment as an effective oral, parenteral or intravenous dose of from 0.10 to 50 mg/kg of body weight per day. A particularly effective amount is about 10 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.
Acetylcholinesterase inhibitors and clonidine binding inhibitors are known in the art as being useful as relievers of memory dysfunction and as antidepressants, respectively. (See V. Kumar in Alzheimer's Disease: Therapeutic Strategies, E. Giacobini and R. Becker Eds.; Birkhauser, Boston 1994 for memory dysfunction utility and K. F. Tipton in Biochemical and Pharmacological Aspects of Depression, K. F. Tipton and U.B.H. Youdin, Eds., Taylor and Francis, London 1989, for antidepressant utility.
Depression frequently attends memory dysfunction associated with Alzheimer's disease and responds to antidepressant intervention. Thus, the antidepressant component of the pharmacological properties of the compounds of

the present invention provide both desirable effects in one chemical entity, providing both therapies in one administration, where indicated. See, for example, W. W. Pendlebury and P. R. Solomon, Neurobiology of Aging, 15, 287 (1994) at page 287, among others.

Effective amounts of the compounds of the invention may be administered to a subject by any one of various methods, for example, orally as in capsules or tablets, parenterally in the form of sterile solutions or suspensions, and in some cases intravenously in the form of sterile solutions. The free base final products, while effective themselves, may be formulated and administered in the form of their pharmaceutically acceptable addition salts for purposes of stability, convenience of crystallization, increased solubility and the like.

Preferred pharmaceutically acceptable addition salts include salts of mineral acids, for example, hydrochloric acid, sulfuric acid, nitric acid and the like, salts of monobasic carboxylic acids such as, for example, acetic acid, propionic acid and the like, salts of dibasic carboxylic acids such as, for example, maleic acid, fumaric acid, oxalic acid and the like, and salts of tribasic carboxylic acids such as, for example, carboxysuccinic acid, citric acid and the like.

The active compounds of the present invention may be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers, chewing gums and the like. These preparations should contain at least 0.5% of active compounds, but may be varied depending upon the particular form and may conveniently be between 4% to about 75% of the weight of the unit. The amount of present compound in such composition is such that a suitable dosage will be obtained. Preferred compositions and preparations according to the present invention are prepared so that an oral dosage unit form contains between 1.0-300 mgs of active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel, com starch and the like; a lubricant such as magnesium stearate or Sterotes; a glidant such as colloidal silicon dioxide; and a sweetening agent such as sucrose or saccharin or a flavoring agent such as peppermint, methyl, salicylate, or orange flavoring may be added. When the dosage unit is a capsule it may contain, in addition to materials of the above type, a liquid carrier such as fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1 % of the aforesaid compound, but may be varied between 0.5 and about 50% of the weight thereof. The amount of active compound in such compositions is such that a suitable dosage will be obtained. Preferred compositions and preparations according to the present invention are prepared so that a parenteral dosage unit contains between 0.5 to 100 mgs of the active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or thyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparations can be enclosed in ampules, disposable syringes or multiple vials made of glass or plastic.

The following examples are for illustrative purposes only and are not to be construed as limiting the invention in any way whatsoever.

## EXAMPLE 9

**4-Methoxy-3-propargyloxy-1-[(4-methylpiperazin-1-yl)methyl]benzene dihydrochloride**

To a solution of 4-methoxy-3-propargyloxybenzaldehyde (19.9 g) in 1,2-dichloroethane (210 ml) was added 1-methylpiperazine (11.7 ml), followed by 1,2-dichloroethane (210 ml) and sodium triacetoxyborohydride (32.5 g). The reaction mixture was stirred at ambient temperature for 2 hrs, poured into 10% sodium hydroxide/ice (1000 ml) and extracted with chloroform. The combined organic extracts were washed with water , dried over anhydrous sodium sulfate, filtered, and the filtrate was concentrated. The residue was dissolved in chloroform and high performance liquid chromatographed, eluting with chloroform, followed by 2%, 4%, 5% and 10% methanol:chloroform. The appropriate fractions were collected and combined to give 15.8 g (52.3%) of product, as the free base. The free base was dissolved in diethyl ether and ethereal hydrogen chloride was added. The precipitate was collected to give product, mp 220-223°C (dec).

| Analysis: | | | |
|---|---|---|---|
| Calculated for $C_{16}H_{24}N_2O_2$: | 55.34%C | 6.97%H | 8.07%N |
| Found: | 55.24%C | 7.01%H | 7.91%N |

**EXAMPLE 11**

**4-Methanesulfonyloxy-3-(propargyloxy)benzaldehyde**

To a stirred suspension of sodium hydride (2.52 g) in dry dimethylformamide (56 ml) at a water bath temperature of 20°C was added, dropwise, a solution of 3-hydroxy-4-methanesulfonyloxybenzaldehyde (14.0 g) in dimethylformamide (56 ml) at a rate such that the reaction temperature remained below 25°C. The reaction mixture was stirred for 5 mins, cooled in an ice/salt bath to 0-5°C, and a solution of propargyl bromide (7 ml) in dimethylformamide (56 ml) was added dropwise. The mixture was stirred at 0-5° for 0.5 hr, poured into ice/water and extracted with chloroform. The layers were separated, sodium carbonate was added to the aqueous phase and the aqueous phase was extracted with chloroform. The combined chloroform extracts were dried over anhydrous sodium sulfate, filtered, and the filtrate was concentrated. The residue was dissolved in ethyl acetate, washed with cold 10% sodium hydroxide solution, filtered and concentrated. The residue was dried at 80°C under high vacuum to give 7.0 g (43.8%) of product. Recrystallization from ethanol gave the analytical sample, mp 81-83°C.

| Analysis: | | |
|---|---|---|
| Calculated for: $C_{11}H_{10}O_5S$: | 51.96%C | 3.96%H |
| Found: | 51.80%C | 3.81%H |

**EXAMPLE 12**

**4-[3-[2-Methoxy-5-(4-methylpiperazin-1-ylmethyl)phenoxy]prop-1-ynyl]tetrahydrothiopyran-4-ol dihydrochloride dihydrate**

To a solution of 4-methoxy-3-propargyloxy-1-[(4-methylpiperazin-1-yl)methyl]benzene (5.69 g) in dry tetrahydrofuran (25 ml) at 0° to 5°C was added 2.2 M n-butyllithium (9.50 ml) over 30 mins at a rate such that the internal temperature remained below 0°C. The reaction mixture was stirred at 0°C for 20 mins, chilled to -30° to -35°C, and after stirring at this temperature for 20 mins, tetrahydrothiopyran-4-one (2.29 g) in dry tetrahydrofuran (25 ml) was added over 15 mins. The reaction mixture was stirred at -30° to -35°C for 30 mins, poured into ice/water (250 ml), and the mixture was extracted with chloroform. The combined organic extracts were dried over anhydrous sodium sulfate, filtered, and the filtrate was concentrated. The residue was dissolved in methylene chloride and high performance liquid chromatographed, eluting with methylene chloride (with a trace of ammonium hydroxide), followed by 2.5% methanol:methylene chloride (with a trace of ammonium hydroxide). The appropriate fractions were collected and concentrated to give 4.37 g (52%) of product, as the free base. The free base was dissolved in ether and ethereal hydrogen chloride was added. The precipitate was collected to give product, mp 230-232°C (dec).

| Analysis: | | | |
|---|---|---|---|
| Calculated for $C_{21}H_{36}Cl_2N_2O_5S$: | 50.50%C | 7.26%H | 5.61%N |
| Found: | 50.97%C | 7.12%H | 5.52%N |

**EXAMPLE 27**

**4-Methoxy-3-(propargyloxy)-1-[[4-(pyridin-2-yl)piperazin-1-yl]methyl] benzene hydrochloride monohydrate**

To a solution of 4-methoxy-3-(propargyloxy)benzaldehyde (1.02 g) in dry 1,2-dichloroethane (22 ml) was added 1-(2-pyridyl)piperazine (0.80 ml), with stirring, and the solution was stirred at ambient temperature for 10 mins. Sodium triacetoxyborohydride (1.73 g) was added, and the reaction mixture was stirred at ambient temperature for 3 hrs, poured into 10% sodium hydroxide/ice and extracted with dichloromethane. The organic extracts were combined, washed with water, dried over anhydrous sodium sulfate, filtered, and the filtrate was concentrated. The residue was dissolved in dichloromethane and flash chromatographed (silica gel), eluting with 3% methanol:dichloromethane followed by 4% methanol:dichloromethane. The appropriate fractions were collected and concentrated to give 1.41 g (78%) of product,

as the free base. The free base was dissolved in diethyl ether and ethereal hydrogen chloride was added. The precipitate was collected to give product, mp 175-185°C.

| Analysis: | | | |
|---|---|---|---|
| Calculated for $C_{20}H_{26}ClN_3O_3$: | 61.30%C | 6.69%H | 10.72%N |
| Found: | 61.63%C | 6.45%H | 10.54%N |

**EXAMPLE 30**

**3-[2-Methoxy-5-[[4-(pyridin-2-yl)piperazin-1-yl]methyl]phenoxy(prop -1-ynyl)tetrahydrothiopyran-4-ol hydrochloride monohydrate**

To a solution of 4-methoxy-3-(propargyloxy)-1-[[4-(pyridin-2-yl)-piperazin-1-yl]methyl]benzene (1.81 g) dissolved in tetrahydrofuran (7.0 ml) at 0 to -5°C was added 2.2 M n-butyllithium (2.4 ml), with stirring. The reaction mixture was stirred at 0 to -5°C for 1 hr, cooled to -30 to -40°C and stirred at -30° to -40°C for 30 mins. Tetrahydrofuran-4-one (0.58 g) dissolved in tetrahydrofuran (7.0 ml) was added via syringe, and the mixture was stirred at -30 to -35°C for 1.5 hr. The reaction mixture was poured into ice/water (50 ml) and extracted with chloroform. The organic extracts were combined, dried over anhydrous sodium sulfate, filtered, and the filtrate was concentrated. The residue was dissolved in dichloromethane and flash chromatographed (silica gel), eluting with dichloromethane, followed by 1, 2, 3, and 5% methanol:dichloromethane. The appropriate fractions were collected and concentrated to give 0.65 g (28%) of product, as the free base. The free base was dissolved in dichloromethane/diethyl ether and ethereal hydrogen chloride was added. The precipitate was collected to provide product, mp 190-201°C.

| Analysis: | | | |
|---|---|---|---|
| Calculated for $C_{25}H_{34}ClN_3O_4S$: | 59.10%C | 6.75%H | 8.27%N |
| Found: | 58.99%C | 6.48%H | 8.09%N |

## Claims

**1.** A compound of the formula

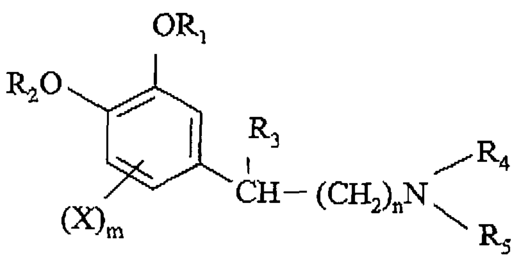

wherein:

$R_1$ is a group of the formula $CH_2C\equiv C\text{-}R_9$;
$R_2$ is hydrogen, $(C_1\text{-}C_{10})$alkyl, a group of the formula $CONR_6R_7$, a group of the formula $SO_2R_{10}$, a group of the formula

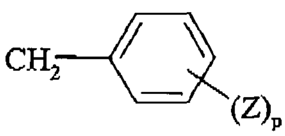

or a group of the formula $Si(R_{11})_3$;

$R_3$ is hydrogen or $(C_1-C_{10})$alkyl;

$R_4$ and $R_5$ taken together with the nitrogen atom to which they are attached form a group of the formula

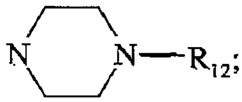

$R_6$ is hydrogen or $(C_1-C_{10})$alkyl;

$R_7$ is $(C_1-C_{10})$alkyl, a group of the formula

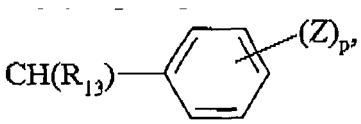

a group of the formula

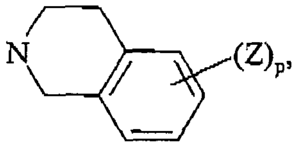

or a group of the formula

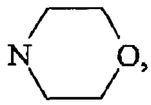

or $R_6$ and $R_7$ taken together with the nitrogen atom to which they are attached form a group of the formula

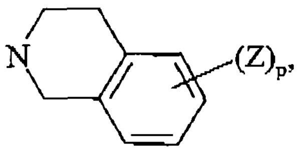

$R_9$ is hydrogen, a group of the formula $C(R_{14}R_{14'})OH$, a group of the formula

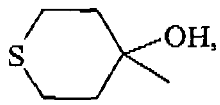

or a group of the formula

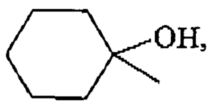

$R_{10}$ is $(C_1\text{-}C_{10})$alkyl;
$R_{11}$ is $(C_1\text{-}C_{10})$alkyl;
$R_{12}$ is $(C_1\text{-}C_{10})$alkyl, hydroxy$(C_1\text{-}C_{10})$alkyl, a group of the formula $COR_{15}$, a group of the formula

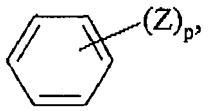

a group of the formula

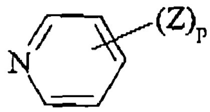

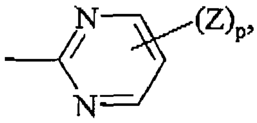

a group of the formula

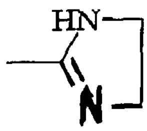

or a group of the formula

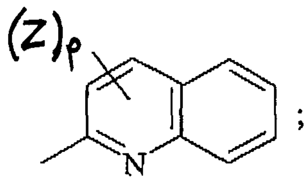

$R_{13}$ is hydrogen or $(C_1\text{-}C_{10})$alkyl;
$R_{14}$ is hydrogen or $(C_1\text{-}C_{10})$alkyl;
$R_{14'}$ is hydrogen or $(C_1\text{-}C_{10})$alkyl;
$R_{15}$ is a group of the formula

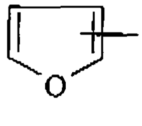

or $(C_1\text{-}C_{10})$alkyl;
$R_{20}$ is $(C_1\text{-}C_{10})$alkyl;
X is hydrogen or halogen;
Z is hydrogen, halogen, $(C_1\text{-}C_{10})$alkyl, hydroxyl, $(C_1\text{-}C_{10})$alkoxy, trifluoromethyl, nitro or cyano, $R_{20}$ CO, or a group of the formula $OCONR_6R_7$;

m is 1 or 2;
n is 0 or 1;
p is 1 or 2;

the optical isomers thereof; or the pharmaceutically acceptable salts thereof.

**2.** A compound according to claim 1 wherein $R_2$ is $(C_1-C_{10})$alkyl.

**3.** A compound according to claim 1 wherein $R_7$ is a group of the formula $CONR_6R_7$.

**4.** A compound according to claim 3 wherein $R_4$ and $R_5$ taken together with the nitrogen to which they are attached form a group of the formula

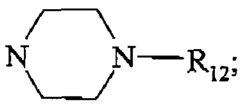

an n is 0.

**5.** A compound according to claim 1 which is selected from: 4-methoxy-3-propargyloxy-1-[(4-methylpiperazin-1-yl) methyl]benzene;
4-[3-[2-methoxy-5-(4-methylpiperazin-1-yl-methyl)phenoxy]prop-1-ynyl]tetrahydrothiopyran-4-ol;
4-methoxy-3-(propargyloxy)-1-[[4-(pyridin-2-yl)piperazin-1-yl]methyl]benzene;
3-[2-methoxy-5-[[4-(pyridin-2-yl)piperazin-1-yl]methyl]phenoxy(prop-1-ynyl)tetrahydrothiopyran-4-ol.

**6.** A compound of any one of claims 1 to 5 for use as a medicament.

**7.** Use of a compound of any one of claims 1 to 5 for preparing a medicament for relieving memory dysfunction in mammals.

**8.** Use of a compound of any one of claims 1 to 5 for preparing a medicament for simultaneously treating depression and memory dysfunction in mammals.

**9.** A pharmaceutical composition containing a compound of any one of claims 1 to 5in admixture with a pharmaceutically acceptable carrier.

**10.** A composition according to claim 9 for relieving memory dysfunction in mammals.

**11.** A composition according to claim 9 for simultaneously treating depression and memory dysfunction in mammals.

## Patentansprüche

**1.** Verbindung der Formel

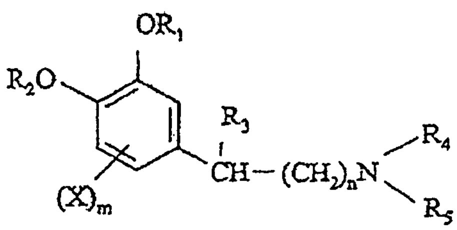

in welcher:

$R_1$ für eine Gruppe der Formel $CH_2C{\equiv}C\text{-}R_9$ steht;
$R_2$ für Wasserstoff, $(C_1\text{-}C_{10})$-Alkyl, eine Gruppe der Formel $CONR_6R_7$, eine Gruppe der Formel $SO_2R_{10}$, eine Gruppe der Formel

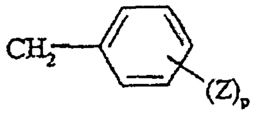

oder eine Gruppe der Formel $Si(R_{11})_3$ steht;
$R_3$ für Wasserstoff oder $(C_1\text{-}C_{10})$-Alkyl steht;
$R_4$ und $R_5$ zusammen mit dem Stickstoffatom, an das sie gebunden sind, eine Gruppe der Formel

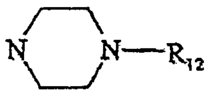

bilden;
$R_6$ für Wasserstoff oder $(C_1\text{-}C_{10})$-Alkyl steht;
$R_7$ für $(C_1\text{-}C_{10})$-Alkyl, eine Gruppe der Formel

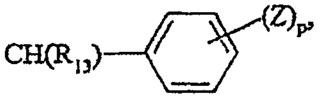

,

eine Gruppe der Formel

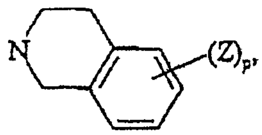

oder eine Gruppe der Formel

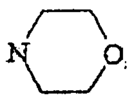

steht,
oder $R_6$ und $R_7$ zusammen mit dem Stickstoffatom, an das sie gebunden sind, eine Gruppe der Formel

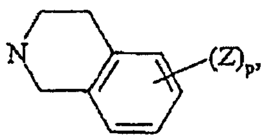

bilden,
$R_9$ für Wasserstoff, eine Gruppe der Formel $C(R_{14}R_{14'})OH$, eine Gruppe der Formel

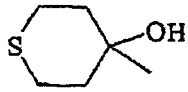

oder eine Gruppe der Formel

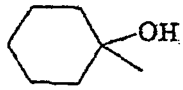

steht,
$R_{10}$ für ($C_1$-$C_{10}$)-Alkyl steht;
$R_{11}$ für ($C_1$-$C_{10}$)-Alkyl steht;
$R_{12}$ für ($C_1$-$C_{10}$) -Alkyl, Hydroxy- ($C_1$-$C_{10}$) -Alkyl, eine Gruppe der Formel $COR_{15}$, eine Gruppe der Formel

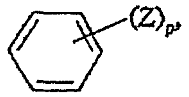

eine Gruppe der Formel

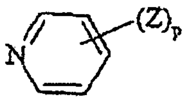

,

eine Gruppe der Formel

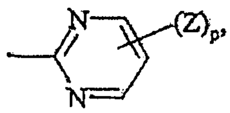

,

eine Gruppe der Formel

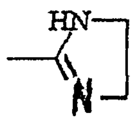

oder eine Gruppe der Formel

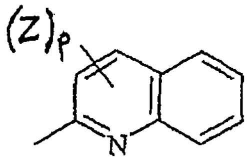

steht;
$R_{13}$ für Wasserstoff oder ($C_1$-$C_{10}$)-Alkyl steht;

$R_{14}$ für Wasserstoff oder ($C_1$-$C_{10}$)-Alkyl steht;
$R_{14'}$ für Wasserstoff oder ($C_1$-$C_{10}$)-Alkyl steht;
$R_{15}$ für eine Gruppe der Formel

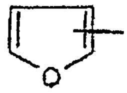

oder ($C_1$-$C_{10}$)-Alkyl steht;
$R_{20}$ für ($C_1$-$C_{10}$)-Alkyl steht;
X für Wasserstoff oder Halogen steht;
Z für Wasserstoff, Halogen, ($C_1$-$C_{10}$)-Alkyl, Hydroxyl, ($C_1$-$C_{10}$)-Alkoxy, Trifluormethyl, Nitro oder Cyano, $R_{20}$CO, oder eine Gruppe der Formel $OCONR_6R_7$ steht;
m für 1 oder 2 steht;
n für 0 oder 1 steht;
p für 1 oder 2 steht;

deren optische Isomere oder deren pharmazeutisch unbedenkliche Salze.

**2.** Verbindung nach Anspruch 1, in welcher $R_2$ für ($C_1$-$C_{10}$) -Alkyl steht.

**3.** Verbindung nach Anspruch 1, in welcher $R_2$ für eine Gruppe der Formel $CONR_6R_7$ steht.

**4.** Verbindung nach Anspruch 3, in welcher $R_4$ und $R_5$ zusammen mit dem Stickstoffatom, an das sie gebunden sind, eine Gruppe der Formel

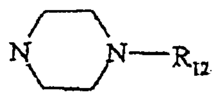

bilden;
und n für 0 steht.

**5.** Verbindung nach Anspruch 1, ausgewählt aus:

4-Methoxy-3-propargyloxy-1-[(4-methylpiperazin-1-yl)methyl] benzol;
4-[3-[2-Methoxy-5-(4-methylpiperazin-1-yl-methyl)phenoxy]prop-1-inyl]tetrahydrothiopyran-4-ol;
4-Methoxy-3-(propargyloxy)-1-[[4-(pyridin-2-yl)piperazin-1-yl]methyl]benzol;
3-[2-Methoxy-5-[[4-(pyridin-2-yl)piperazin-1-yl]methyl]phenoxy(prop-1-inyl)tetrahydrothiopyran-4-ol.

**6.** Verbindung nach einem der Ansprüche 1 bis 5 zur Verwendung als Medikament.

**7.** Verwendung einer Verbindung nach einem der Ansprüche 1 bis 5 zur Herstellung eines Medikaments zur Linderung von Gedächtnisstörungen in Säugetieren.

**8.** Verwendung einer Verbindung nach einem der Ansprüche 1 bis 5 zur Herstellung eines Medikaments zur gleichzeitigen Behandlung von Depression und Gedächtnisstörungen in Säugetieren.

**9.** Pharmazeutische Zusammensetzung, enthaltend eine Verbindung nach einem der Ansprüche 1 bis 5 in einer Mischung mit einem pharmazeutisch unbedenklichen Träger.

**10.** Zusammensetzung nach Anspruch 9 zur Linderung von Gedächtnisstörungen in Säugetieren.

**11.** Zusammensetzung nach Anspruch 9 zur gleichzeitigen Behandlung von Depression und Gedächtnisstörungen in Säugetieren.

## Revendications

**1.** Composé de la formule

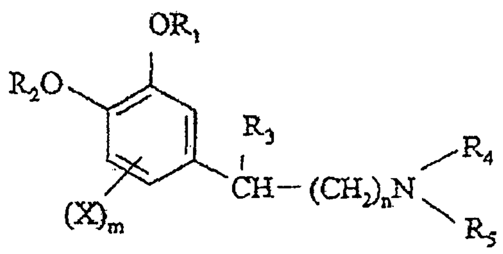

dans laquelle :

$R_1$ est un groupe de la formule $CH_2C{\equiv}C\text{-}R_9$ ;
$R_2$ est un atome d'hydrogène, un groupe alkyle (en $C_1$ à $C_{10}$), un groupe de la formule $CONR_6R_7$, un groupe de la formule $SO_2R_{10}$, un groupe de la formule

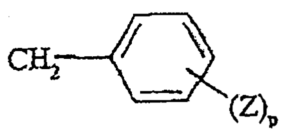

ou un groupe de la formule $Si(R_{11})_3$ ;
$R_3$ est un atome d'hydrogène ou un groupe alkyle (en $C_1$ à $C_{10}$) ;
$R_4$ et $R_5$ pris ensemble avec l'atome d'azote auquel ils sont rattachés forment un groupe de la formule

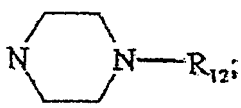

$R_6$ est un atome d'hydrogène ou un groupe alkyle (en $C_1$ à $C_{10}$) ;
$R_7$ est un groupe alkyle (en $C_1$ à $C_{10}$), un groupe de la formule

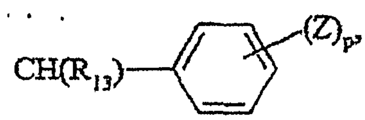

un groupe de la formule

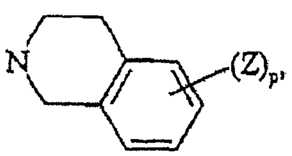

ou un groupe de la formule

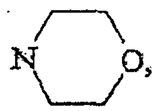

ou $R_6$ et $R_7$ pris ensemble avec l'atome d'azote auquel ils sont rattachés forment un groupe de la formule

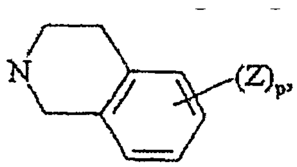

$R_9$ est un atome d'hydrogène, un groupe de la formule $C(R_{14}R_{14'})OH$, un groupe de la formule

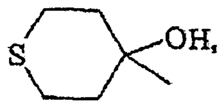

ou un groupe de la formule

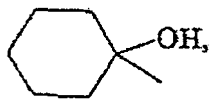

$R_{10}$ est un groupe alkyle (en $C_1$ à $C_{10}$) ;
$R_{11}$ est un groupe alkyle (en $C_1$ à $C_{10}$) ;
$R_{12}$ est un groupe alkyle (en $C_1$ à $C_{10}$), un groupe hydroxyalkyle (en $C_1$ à $C_{10}$), un groupe de la formule $COR_{15}$, un groupe de la formule

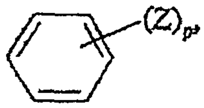

un groupe de la formule

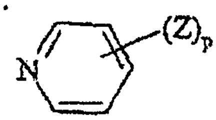

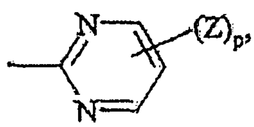

un groupe de la formule

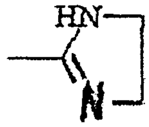

ou un groupe de la formule

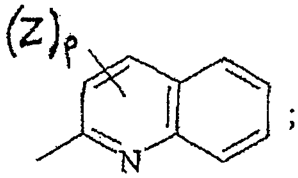

$R_{13}$ est un atome d'hydrogène ou un groupe alkyle (en $C_1$ à $C_{10}$) ;
$R_{14}$ est un atome d'hydrogène ou un groupe alkyle (en $C_1$ à $C_{10}$) ;
$R_{14}$ est un atome d'hydrogène ou un groupe alkyle (en $C_1$ à $C_{10}$) ;
$R_{15}$ est un groupe de la formule

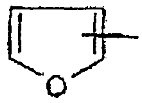

ou un groupe alkyle (en $C_1$ à $C_{10}$) ;
$R_{20}$ est un groupe alkyle (en $C_1$ à $C_{10}$) ;
X est un atome d'hydrogène ou un halogène,
Z est un atome d'hydrogène, un halogène, un groupe alkyle (en $C_1$ à $C_{10}$), un groupe hydroxyle, un groupe alcoxy en (en $C_1$ à $C_{10}$), un groupe trifluorométhyle, un groupe nitro ou cyano, un groupe $R_{20}$ CO, ou un groupe de la formule $OCONR_6R_7$ ;
m vaut 1 ou 2 ;
n vaut 0 ou 1 ;
p vaut 1 ou 2 ;

les isomères optiques de ceux-ci, ou des sels pharmaceutiquement acceptables de ceux-ci.

**2.** Composé selon la revendication 1, dans lequel $R_2$ est un groupe alkyle (en $C_1$ à $C_{10}$).

**3.** Composé selon la revendication 1, dans lequel $R_2$ est un groupe de la formule $CONR_6R_7$.

**4.** Composé selon la revendication 3, dans lequel $R_4$ et $R_5$ pris ensemble avec l'atome d'azote auquel ils sont rattachés forment un groupe de la formule

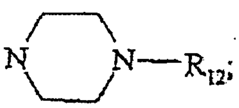

et n vaut 0.

**5.** Composé selon la revendication 1, lequel est choisi partir du groupe suivant :

4-méthoxy-3-propargyloxy-1-[(4-méthylpipérazin-1-yl)méthyl]benzène ;
4-[3-[2-méthoxy-5-(4-méthylpipérazin-1-yl-méthyl)phénoxy]prop-1-ynyl]tétrahydrothiopyran-4-ol ;

4-méthoxy-3-(propargyloxy)-1-[[4-(pyridine-2-yl)pipérazin-1-yl]méthyl]benzène ;
3-[2-méthoxy-5-[[4-(pyridine-2-yl)pipérazin-1-yl]méthyl]phénoxy(prop-1-ynyl)tétrahydrothiopyran-4-ol.

**6.** Composé selon l'une quelconque des revendications 1 à 5 pour utilisation comme médicament.

**7.** Utilisation d'un composé selon l'une quelconque des revendications 1 à 5 pour la préparation d'un médicament pour le soulagement du dysfonctionnement de la mémoire chez des mammifères.

**8.** Utilisation d'un composé selon l'une quelconque des revendications 1 à 5 pour la préparation d'un médicament pour le traitement simultané de la dépression et du dysfonctionnement de la mémoire chez des mammifères.

**9.** Composition pharmaceutique contenant un composé selon l'une quelconque des revendications 1 à 5 dans un mélange avec un vecteur pharmaceutiquement acceptable.

**10.** Composition selon la revendication 9 pour le soulagement du dysfonctionnement de la mémoire chez des mammifères.

**11.** Composition selon la revendication 9 pour le traitement simultané de la dépression et du dysfonctionnement de la mémoire chez les mammifères.